# EUROPÄISCHE PATENTSCHRIFT

(11) **EP 1 597 271 B1**
(45) Veröffentlichungstag und Bekanntmachung des Hinweises auf die Patenterteilung: **06.03.2013**
(21) Anmeldenummer: 04706129.6
(22) Anmeldetag: 29.01.2004
(51) Int. Cl.: C07K 7/06, C12N 15/11, C12N 15/12, C07K 16/18, A61K 38/03

(54) **PEPTIDE GEGEN KÄLTEUNVERTRÄGLICHKEIT HERVORRUFENDE AUTOANTIKÖRPER UND IHRE VERWENDUNG**
PEPTIDES DIRECTED AGAINST ANTIBODIES, WHICH CAUSE COLD-INTOLERANCE, AND THE USE THEREOF
PEPTIDES DIRIGES CONTRE DES AUTOANTICORPS RESULTANT DE L'INTOLERANCE AU FROID, ET LEUR UTILISATION

(30) Priorität: 31.01.2003 DE 10305165; 06.03.2003 DE 10311106
(43) Veröffentlichungstag der Anmeldung: 23.11.2005
(73) Patentinhaber: MAX-DELBRÜCK-CENTRUM FÜR MOLEKULARE MEDIZIN, 13125 Berlin (DE)
(72) Erfinder: WALLUKAT, Gerd, 13129 Berlin (DE); HARRER, Thomas, 91054 Erlangen (DE)
(74) Vertreter: Spieker, Oliver
(86) Internationale Anmeldenummer: PCT/DE2004/000169
(87) Internationale Veröffentlichungsnummer: WO 2004/067549

(56) Entgegenhaltungen:
- WO-A-2004/051280
- JP-A- 2002 010 784
- DATABASE EMBL 6. April 1991 (1991-04-06), "Human thrombin receptor mRNA; complete cds" XP002290141 Database accession no. M62424 -& DATABASE UNIPROT 1. Mai 1992 (1992-05-01), "Proteinase activated receptor 1 precursor (PAR-1) (Thrombin receptor) (Coagulation factor II receptor)" XP002290142 Database accession no. P25116
- DATABASE UNIPROT 1. Oktober 1996 (1996-10-01), "Proteinase activated receptor 2 precursor (PAR-2) (Thrombin receptor-like 1)(Coagulation factor II receptor-like 1)" XP002290143 Database accession no. P55085
- DATABASE UNIPROT 15. Juli 1998 (1998-07-15), "Proteinase activated receptor 3 precursor (PAR-3) (Thrombin receptor-like 2) (Coagulation factor II receptor-like 2)" XP002290144 Database accession no. O00254
- MACFARLANE S R ET AL: "Proteinase-activated receptors" PHARMACOLOGICAL REVIEWS, WILLIAMS AND WILKINS INC., BALTIMORE, MD,, US, Bd. 53, Nr. 2, Juni 2001 (2001-06), Seiten 245-282, XP002234540 ISSN: 0031-6997
- TAKAMI: "Genome sequence of Oceanobacillus iheyensis isolated from the Iheya Ridge and its unexpected adaptive capabilities to extreme environments", NUCLEIC ACIDS RESEARCH, vol. 30, no. 18, 2002,
- DATABASE REGISTRY [Online] CHEMICAL ABSTRACTS SERVICE, COLUMBUS, OHIO, US Database accession no. 475319-02-9

## Beschreibung

Die Erfindung betrifft Peptide, die mit Autoantikörper wechselwirken, die mit der Kälteallergie assoziiert sind, eine umfassend die sowie die ex vivo Verwendung der Peptide - insbesondere in der Apherese - zur Behandlung von mit Kälteexpositionen bzw. Kälteunverträglichkeit in Zusammenhang stehenden Durchblutungsstörungen, besonders von Kälteallergien.

Mehrzellige Organismen, insbesondere Säugetiere, und vor allem der Mensch, setzen sich mit ihrer Umwelt über verschiedene biochemische und/oder immunologische Regelmechanismen auseinander. Ein wichtiger biochemischer Regelmechanismus ist beispielsweise der, der für das Konstanthalten einer bestimmten inneren Temperatur im Organismus verantwortlich ist. Ein weiterer wichtiger Regelmechanismus stellt zelluläre und humorale Substanzen, wie zum Beispiel Antikörper, zur Verfügung, die dem Organismus die Möglichkeit geben, sich mit Mikroorganismen,. Parasiten, aber auch Tumoren bzw. andere Erkrankungen erfolgreich auseinander zu setzen. Die Funktion der Antikörper beruht vor allem darauf, dass diese "fremd" und "selbst" unterscheiden können. Das heißt, der Organismus produziert selbst Antikörper, die so ausgebildet sind, dass sie körpereigene Strukturen, wie Gewebe oder Organe, nicht attackieren, aber an Bakterien, Parasiten oder andere körperfremde Bestandteile binden und so immunologische Reaktionen initiieren bzw. unterstützen.

Bei der biochemischen Entstehung der Antikörperantwort im Organismus kommt es zu somatischen Hypermutationen von Antikörper produzierenden B-Zellen während einer T-Zellenabhängigen Immunantwort. Diese Mutationen betreffen selektiv die Immunglobulingene und sind wesentlich für die Bildung von Vorläuferzellen für Antikörper bildende Zellen mit hoher Affinität. In diesem Zusammenhang handelt es sich bei der Mutation um einen normalen und im wesentlichen nicht pathogenen Vorgang. Mutationen können aber auch zur Bildung von hochaffinen Autoantikörpern führen und somit den Organismus schädigen.

Autoantikörper sind mit der Ausprägung von Autoimmunkrankheiten eng assoziiert oder für diese ursächlich verantwortlich. Autoimmunkrankheiten sind Allergiekrankheiten, bei denen Autoantikörper und/oder Autoimmunzellen detektiert werden können, wobei derzeit nicht bekannt ist, wie es zur Entstehung der Autoimmunkrankheiten kommt und ob die Autoantikörper die alleinige Ursache, ein Ergebnis oder nur eine Begleiterscheinung der Erkrankung sind.

Bei zahlreichen Autoimmunkrankheiten, wie zum Beispiel der Kälteallergie, ist jedoch bekannt, dass Autoantikörper für das Entstehungsbild der Erkrankung verantwortlich sind.

Auch Krankheiten wie Rheuma oder Lupus erythematodes werden zu den Antikörper vermittelten Autoimmunkrankheiten gezählt. Zahlreiche - und für die Betroffenen sehr nachteilige Autoimmunkrankheiten -, betreffen wichtige biochemische Kreisläufe im Organismus, beispielsweise die Wärmeregulation. Das heißt, durch das Auftreten von Autoantikörpern kommt es zu örtlichen oder allgemeinen Schädigungen des Wärmehaushaltes des Organismus, was zu Erfrierungen, zur Kälteunverträglichkeit, zur kältebedingten Endothelschädigung, zur Quellung, zur Thrombenbildung, Nervendegeneration bzw. zu Muskel-, Fettgewebs-, und/oder Knochennekrosen führen kann. Zahlreichen dieser Erkrankungen ist gemeinsam, dass es zu Durchblutungsstörungen an Körperenden, wie beispielsweise den Füßen, den Händen oder auch den Ohren bzw. sekundären Geschlechtsmerkmalen kommt. Beispiele für derartige Erkrankungen sind die Akrozyanose, die Kältehämagglutinationskrankheit, das Hyperviskositätssyndrom, das Ischämiesyndrom, die Akrotrophoneurose, die Sklerodermie, das Kälteerythem, die Kältepurpura, die Kälteurtikaria, die Kryopathie, die Kryoglobulinämie und besonders die Kälteallergie und insbesondere das Raynaud-Syndrom.

Derartige Krankheiten treten mit hoher Häufigkeit in der Bevölkerung auf. Allein das Raynaud-Syndrom tritt bei 3 bis 16 % der Bevölkerung auf, wobei frauen 5 bis 10-mal häufiger davon betroffen sind als Männer. Die ersten Symptome beginnen sich typischerweise im Alter von 14 bis 40 Jahren bemerkbar zu machen, wobei Männer in späteren Lebensabschnitten betroffen sind. Das Fehlen einer effektiven Therapie ist unter anderem auch deshalb nachteilig, da bereits das Hineinfassen in einen Kühlschrank oder in eine Tiefkühltruhe, ein Griff an ein kaltes Lenkrad oder auch Händewaschen mit kaltem Wasser ausreicht, um beispielsweise eine solche Durchblutungsstörung, insbesondere das Raynaud-Syndrom, auszulösen. In vielen derartigen Lebensabläufen ist es den handelnden Personen nur bedingt möglich, die Kälte zu meiden.

Bei dem Raynaud-Syndrom ist die Blutversorgung der Gliedmaßen, meistens der Finger und der Zehen, aber manchmal auch der Ohren und der Nase, vorübergehend unterbrochen. Während eines Anfalls werden die betroffenen Gliedmaßen zuerst weiß und werden optisch in der Regel als bereits abgestorben wahrgenommen, dann werden sie blau und schließlich rot, verbunden mit einem brennenden Gefühl. Diese Autoimmunreaktion ist von beträchtlichem Schmerz und wird von Taubheitsgefühl und Kribbeln begleitet. Eine effektive und sichere Diagnose oder Therapie kann für diese Kälteassoziierten pathogenen Veränderungen derzeit für die Betroffenen nur sehr bedingt angeboten werden. Im Allgemeinen wird den betroffenen Personen empfohlen, Kälteexpositionen der Extremitäten zu vermeiden bzw. ein sofortiges Aufwärmen einzuleiten, sofern Vasospasmen bereits eingetreten sind. Weiterhin ist es möglich, durch lokales Aufsprühen von Glyceroltrinitrat einige Phänomene der Krankheit zu behandeln. Zum Teil wurden auch Erfolge durch die Gabe von Calciumantagonisten berichtet. Als ultima ratio wird das Ausschalten von Ganglien durch Sympathektomie empfohlen. Diese gezielte Nervendurchtrennung führt dazu, dass der Patient vorübergehend zumindest für einige Monate oder auch Jahre beschwerdefrei ist. Seit kurzem ist es möglich, mit einem bestimmten Rotlicht-Laser das Raynaud-Syndrom positiv zu beeinflussen. Nachteilig ist jedoch, dass aus Kapazitätsgründen ein routinemäßiger Einsatz dieser neuen Therapiemethode nicht möglich ist.

Aufgabe der Erfindung war es daher, Verbindungen bereitzustellen, mit denen die mit Kälte assoziierten Durchblutungsstörungen, insbesondere das Raynaud-Syndrom, einfach, sicher und effektiv diagnostiziert und therapiert werden können.

Die Erfindung löst dieses technische Problem durch die Bereitstellung eines Peptides - ausgewählt aus der Gruppe bestehend aus
a) einem Peptid bestehend aus der Aminosäuresequenz ITTCHDVL, ITTCHDAL und/oder LNITTCHD, oder
b) einem Peptid gemäß einer Aminosäuresequenz a), welches durch Deletionen, Substitutionen, Translokationen und/oder Inversionen modifiziert ist und die Sequenz des Peptids 60%, vorzugsweise 70%, bevorzugt 80%, ganz besonders bevorzugt 90% homolog zu einer der unter a) angegebenen Aminosäuresequenz ist, wobei
die Peptide mit einen an der zweiten extrazellulären Schleife eines Proteaseaktivierten Rezeptor (PAR), ausgewählt aus der Gruppe umfassend PAR-1, PAR-2 und/oder PAR-3, wechselwirkenden Autoantikörper binden für die ex vivo Verwendung zur Prophylaxe, Diagnose, Therapie, Verlaufskontrolle und/oder Nachbehandlung von mit Kälte-induzierten Autoantikörper-vermittelten Durchblutungsstörungen.

Die Erfindung betrifft demgemäß die überraschende Lehre, dass die erfindungsgemäßen. Peptide bei Missfunktionen der Wärmeregulation und von Durchblutungsstörungen, insbesondere von kleinen Blutgefäßen, die vor allem Hände, Füße, Ohren, Wangen, Nase und Brustwarzen mit Blut versorgen, zur Diagnose und Therapie eingesetzt werden können. Die peptide sind in der Prophylaxe, Diagnose, Therapie, Verlaufskontrolle und/oder Nachbehandlung von Durchblutungsstörungen, insbesondere der Kälteallergie und besonders bevorzugt dem Raynaud-Syndrom, in verschiedenen Formen einsetzbar.

Im Sinne der Erfindung heißt, um zu den genannten Peptid-sequenzen funktionsanalog zu sein, dass die Homologen bei Durchblutungsstörungen, insbesondere der Kälteallergie, ein Verhalten zeigen, dass sie sicher und effektiv bei der Diagnose und/oder der Therapie dieser Erkrankungen bzw. pathogenen Zustände, die mit diesen Krankheiten assoziiert sind, einsetzbar sind. Funktionsanaloge Sequenzen im Sinne der Erfindung sind all jene Sequenzen, die der Fachmann durch Routineversuche als gleichwirkend identifizieren kann.

Das erfindungsgemäße Peptid kann überraschenderweise bei der Diagnose und/oder Therapie von mit Kälteunverträglichkeit im Zusammenhang stehenden Erkrankungen eingesetzt werden. Die erfindungsgemäßen Peptide werden insbesondere von. Autoantikörpern von Patienten gebunden, die an der Akrozyanose, der Kältehämagglutinationskrankheit, dem Hyperviskositätssyndrom, dem Ischämiesyndrom, der Akrotrophoneurose, der Sklerodermie, dem Kälteerythem, der Kältepurpura, der Kälteurtikaria, der Kryopathie und/oder der Kryoglobulinämie, insbesondere der Kälteallergie und besonders bevorzugt dem Raynaud-Syndrom, erkrankt sind.

Der Fachmann kann durch die Offenbarung der erfindungsgemäßen Lehre weitere äquivalente Peptide generieren, die funktionsanalog zu Peptiden mit der Sequenzabfolge: ITTCHDVL, ITTCHDAL oder LNITTCHD bzw. LNITTCHDCL. sind. Natürlich ist es auch möglich, Peptide zu verwenden, die die genannten Strukturen umfassen, wie z.B.. QTIQVPGLNITTCHDVLNETLL oder QTIFIPALNITTCHDVLPEQLL. Aber selbstverständlich sind die erfindungsgemäßen Peptide nicht so ausgewählt, dass sie die natürlich vorkommenden PAR-1, -2 oder -3 Rezeptoren vollständig darstellen. Die erfindungsgemäßen Peptide beziehen sich insbesondere auf ausgewählte Bereiche der zweiten extrazellulären Schleife des PAR-1, -2, oder -3 Rezeptors. Die vorteilhaften Sequenzen ITTCHDVL, ITTCHDAL oder LNITTCHD können aber auch weitere Aminosäuren, die die genannten Sequenzen natürlicherweise in der zweiten extrazellulären Schleife des Rezeptors flankieren, umfassen, ohne jedoch die flankierenden Bereiche so groß zu wählen, dass die Rezeptoren selbst ausgewählt sind. Beispiele für solche - um flankierende oder auch andere artifizielle Bereiche erweiterte - Sequenzen sind z.B.: LNITTCHDCL, QTIQVPGLNITTCHDVLNETLL oder QTIFIPALNITTCHDVLPEQLL.

Die Peptide im Sinne der Erfindung können daher so aufgebaut sein und soviel weitere Aminosäuren, Spacer oder andere Strukturen umfassen, dass sie geeignet sind, mit den Antikörpern zu interagieren, vorzugsweise so, dass sie ein Epitop für diese darstellen.

Die erfindungsgemäßen Peptide sind demgemäss nicht auf die Sequenzen ITTCHDVL, ITTCHDAL und/oder LNITTCHD beschränkt, sondern die Peptide sind bevorzugt Antikörper-Epitope, die im wesentlichen die genannten oder funktionsanaloge Sequenzen enthalten, so dass sie das Epitop in einer Art und Weise darstellen und charakterisieren, dass die Antikörper mit ihnen spezifisch wechselwirken. Die Begriffe Epitop und Peptid können unter bestimmten Bedingungen daher auch synonym verwendet werden.

Es ist weiterhin auch möglich, einzelne oder Gruppen von. Aminosäuren auszutauschen, ohne dass die Aktivität der Peptide in Bezug auf die Lösung der erfindungsgemäßen Aufgabe nachteilig beeinflusst wird. Für den Austausch' derartiger Aminosäuren sei auf die entsprechenden Standardwerke der Biochemie und der Genetik verwiesen.

Im Stand der Technik sind verschiedene Möglichkeiten zur Herstellung von Peptiden offenbart. Peptide, die von den erfindungsgemäßen Peptiden ausgehend mit solchen Verfahren designt werden, sind von der erfindungemäßen Lehre mit erfasst. Eine Möglichkeit des Generierens von funktionsanalogen Peptiden ist beispielsweise in PNAS USA 1998, Oct. 13; 9521:12179-84, WO 99/6293 und/oder WO 02/38592 beschrieben; Das heißt, sämtliche Peptide, Peptidfragmente oder Strukturen, die Peptide umfassen, die mit den genannten Verfahren - vorn den erfindungsgemäßen Peptiden ausgehend - generiert wurden, sind Peptide im Sinne der Erfindung, sofern sie die erfindungsgemäße Aufgabe lösen, insbesondere mit den krankheitsverursachenden Autoantikörpern wechselwirken. Bei diesen Autoantikörpern kann es sich beispielsweise um agonistische Autoantikörper handeln, die Rezeptoren aktivieren. Im folgenden sind bevorzugte dieser Rezeptoren beschrieben.

Die Peptidsequenzen können beispielsweise ein natürlicher Bestandteil der zweiten extrazellulären Schleife des Thrombin-Rezeptors (PAR-1), dem Trypsin- und Tryptase-Rezeptor (PAR-2) und/oder des Thrombin-Rezeptors bzw. Rezeptor unbekannter Pröteasen (PAR-3) sein. Überraschenderweise wechselwirken die Autoantikörper spezifisch mit dem zweiten extrazellulären Loop bzw. der Schleife des jeweiligen Protease-aktivierten Rezeptors (PAR). Die Erfindung betrifft demgemäß auch die überraschende Lehre, däss das Hauptepitop der Autoantikörper, die mit Kälte-induzierten Krankheiten assoziiert sind, insbesondere der Kälteallergie und besonders bevorzugt dem Raynaud-Syndrom, Peptide mit der Aminosäuresequenz ITTCHDVL, ITTCHDAL und/oder LNITTCHD sind. Diese Sequenz ist auf dem PAR-1 und PAR-2 vorzugsweise völlig identisch. Bei PAR-3 ist das nahe dem C-terminalen Ende gelegene Valin (V) durch Alanin (A) ersetzt. Bei den Autoantikörpern kann es sich demgemäß um Antikörper handeln, die als agonistische Autoantikörper fungieren, d.h. diese Autoantikörper sind in der Lage, durch die Bindung an die entsprechenden Rezeptoren diese zu aktivieren. Durch diese Aktivierung des Rezeptors können verschiedene Krankheiten bzw. pathogene Veränderungen induziert und/oder begleitet werden. Beispielsweise ist es möglich, dass durch die Aktivierung der Rezeptoren die Produktion von Messenger-Molekülen moduliert wird. Es ist jedoch auch möglich, dass die Autoantikörper die Rezeptoren der Zellen schädigen, die für eine im wesentlichen normale Durchblutung der Gliedmaßen verantwortlich sind. Im Gegensatz zu der Wirkung der agonistischen Autoantikörper steht bei nicht-agonistischen Autoantikörpern die üblicherweise bei Antikörpern beobachtete Funktion des Attackierens und Schädigens durch chronische Beanspruchung des entsprechenden Targets im Vordergrund; dies schließt jedoch eine Aktivierung oder Deaktivierung der Rezeptoren nicht aus.

In einer bevorzugten Ausführungsform der Erfindung umfasst das Peptid zusätzlich Aminogruppen, Amide, Acetylgruppen, Biotingruppen, Marker, Spacer und/oder Linker.

Vorteilhafterweise ist es möglich, das Peptid durch derartige Strukturen in verschiedenen Bereichen der ex vivo Diagnose und Therapie von Autoimmunkrankheiten einzusetzen. Dem Fachmann sind verschiedene Möglichkeiten bekannt, Peptide für verschiedene Verwendungen zu modifizieren. Es ist dass die Peptide an das Trägermaterial von Affinitätssäulen gebunden werden, um zur Reinigung von Körperflüssigkeiten, insbesondere von Blut, verwendet zu werden, die Bindung der Peptide an eine Matrix erfordert bestimmte strukturelle Modifikationen der erfindungsgemäßen Peptide, die dem Fachmann ebenfalls bekannt oder durch Routineversuche zu ermitteln sind.

Dem Fachmann ist bekannt, dass einzelne Aminosäuren analoge physikochemische Eigenschaften aufweisen, die mit Vorteil dazu führen, dass diese Aminosäuren untereinander ausgetauscht werden können. Hierzu gehören beispielsweise die Gruppe der Aminosäuren (a) Glycin, Alanin, Valin, Leucin und/oder Isoleucin; bzw. die Aminosäuren (b) Serin und Threonin, die Aminosäuren (c) Asparagin und Glutamin, die Aminosäuren (d) Asparaginsäure und Glutaminsäure; die Aminosäuren (e) Lysin und Arginin sowie die Gruppe der aromatischen Aminosäuren (f) Phenylalanin, Tyrosin und/oder Tryptophan. Aminosäuren innerhalb ein und derselben Gruppe (a-f) können untereinander ausgetauscht werden. Weiterhin ist es möglich, dass Aminosäuren durch modifizierte Aminosäuren oder spezifische Enantiomere ausgetauscht werden. Weitere Modifikationen sind gemäß der Lehre nach der WO99/62933 oder WO02/38592-möglich.

In einer weiteren bevorzugten Ausführungsform umfasst das Peptid einen Linker und/oder einen Spacer, der ausgewählt ist aus der Gruppe umfassend: α-Aminocarbonsäuren sowie deren Homo- und Heterooligomere, α,ω-Aminocarbonsäuren sowie deren verzweigte Homo- oder Heterooligomere, sonstige Aminosäuren sowie die linearen und verzweigten Homo- oder Heterooligomere (Peptide); Amino-oligoalkoxy-alkylamine; Maleinimidocarbonsäure-Derivate; Oligomere von Alkylaminen; 4-Alkylphenyl-Derivate; 4-Oligoalkoxyphenyl- oder 4-Oligoalkoxyphenoxy-Derivate; 4-Oligoalkylmercaptophenyl- oder 4-Oligoalkylmercaptophenoxy-Derivate; 4-Oligoalkylaminphenyl- oder 4-Oligoalkylaminyphenoxy-Derivate; (Oligoalkylbenzyl)-phenyl- oder 4-Oligoalkylbenzyl)-phenoxy-Derivate sowie 4-Oligoalkoxybenzyl)-phenyl- oder 4-Oligoalkoxybenzyl)-phenoxy-Derivate; Trityl-Derivate; Benzyloxyaryl- oder Benzyloxyalkyl-Derivate; Xanthen-3-yl-oxyalkyl-Derivate; (4-Alkylphenyl)- oder ω-(4-Alkylphenoxy)-alkansäure-Derivate; Oligoalkyl-Phenoxyalkyl- oder Oligoalkoxyphenoxyalkyl-Derivate; Carbamat-Derivate; Amine; Trialkylsilyl- oder Dialkyl-alkoxysilyl-Derivate; Alkyl- oder Aryl-Derivate und/oder Kombinationen davon; weitere mögliche Strukturen werden in der EP 1 214 350 beschrieben.

Nach einer Ausführungsform der Erfindung ist das Peptid ausgewählt aus der Gruppe bestehend aus

In einer besonderen Ausführungsform der Erfindung ist das Peptid, was eine ausreichende Homologie aufweist, um zu einer der folgenden Aminosäuren ITTCHDVL, ITTCHDAL und/oder LNITTCHD funktionsanalog zu sein, zu diesen zumindest 60% homolog.

In einer weiteren bevorzugten Ausführungsform sind diese Aminosäuresequenzen mindestens 60 %, vorzugsweise 70 %, bevorzugt 80 %, ganz besonders bevorzugt 90 %, homolog zu einer der folgenden Aminosäuresequenzen: ITTCHDVL, ITTCHDAL und/oder LNITTCHD.

In einer ganz besonders bevorzugten Ausführungsform der Erfindung besteht das Peptid im Wesentlichen aus der Aminosäuresequenz ITTCHDVL, ITTCHDAL und/oder LNITTCHD. Insbesondere besteht das Peptid aus Abwandlungen dieser Sequenzen, die nach der WO99/62933 und der WO02/38592 gewonnen wurden, wobei diese Peptide selbstverständlich Autoantikörper im Sinne der Erfindung binden.

In einer weiteren bevorzugten Ausführungsform der Erfindung wird das Peptid als therapeutischer. Wirkstoff ex vivo eingesetzt oder verwendet. Die Verwendung als therapeutischer Wirkstoff meint im Sinne der Erfindung die Anwendung des Peptides auf dem gesamten Gebiet der Medizin.

In einer weiteren besonders bevorzugten Ausführungsform der Erfindung wird das Peptid von spezifischen Antikörpern von Patienten mit einer Kälteallergie gebunden, insbesondere von Autoantikörpern. Bei einem definierten, dem Fachmann bekannten Mengenverhältnis von Peptid und Autoantikörper kommt es zur Ausbildung von Autoantikörper-Peptid-Komplexen, die beispielsweise ausfallen, oder spezifisches Reaktionsverhalten dergestalt zeigen, dass sie zur Eliminierung der Autoantikörper genutzt werden können.

In einer weiteren bevorzugten Ausführungsform der Erfindung ist insbesondere das Peptid immobilisiert. Im Sinne der Erfindung werden unter Immobilisierung verschiedene Verfahren und Techniken zum Fixieren der Peptide auf bestimmten Trägern beispielsweise gemäß der WO99/56126 oder der WO02/26292 verstanden. Die Immobilisierung kann beispielsweise der Stabilisierung der Peptide dienen, wodurch diese insbesondere bei Lagerung oder bei einmaligem Batch-Ansatz durch biologische, chemische oder physikalische Einwirkungen in ihrer Aktivität nicht reduziert oder nachteilig modifiziert werden. Durch die Immobilisierung der Peptide ist ein wiederholter Einsatz unter technischen oder klinischen Routine-Bedingungen möglich; weiterhin kann eine Probe - bevorzugt Blutbestandteile - mit mindestens einem der erfindungsgemäßen Peptide kontinuierlich umgesetzt werden. Dies kann insbesondere durch verschiedene Immobilisierungstechniken erreicht werden, wobei die Bindung der Peptide an andere Peptide oder Moleküle bzw. an einen Träger so erfolgt, dass die dreidimensionale Struktur, insbesondere an dem Zentrum, das die Wechselwirkung mit den Autoantikörpern vermittelt, der entsprechenden Moleküle, insbesondere der Peptid, nicht verändert wird. Vorteilhafterweise geht die Spezifität zu den Autoantikörpern der Patienten durch die Immobilisierung nicht verloren. Im Sinne der Erfindung können drei grundsätzliche Methoden zur Immobilisierung verwendet werden:
(i) Quervernetzung: Bei der Quervernetzung werden die Peptide miteinander fixiert, ohne dass ihre Aktivität nachteilig beeinflusst wird. Sie sind vorteilhafterweise durch die Quervernetzung nicht mehr löslich.
(ii) Bindung an einen Träger: Die Bindung an einen Träger erfolgt zum Beispiel durch Adsorption, Ionenbindung oder kovalente Bindung. Dies kann auch innerhalb von mikrobiellen Zellen bzw. Liposomen oder anderen membranhaltigen geschlossenen bzw. offenen Strukturen erfolgen. Die Peptide werden durch die Fixierung vorteilhafterweise nicht in ihrer Aktivität beeinflusst. Die Peptide können mit Vorteil zum Beispiel in der Klinik in Diagnose oder Therapie trägergebunden mehrfach oder kontinuierlich eingesetzt werden.
(iii) Einschluss: Der Einschluss erfolgt im Sinne der Erfindung insbesondere an eine semipermeable Membran in Form von Gelen, Fibrillen oder Fasern. Gekapselte Peptide sind durch eine semipermeable Membran so durch die umgebende Probenlösung getrennt, dass sie vorteilhafterweise noch mit den Autoantikörpern oder mit Fragmenten dieser interagieren können. Für die Immobilisierung stehen verschiedene Verfahren zur Verfügung, wie beispielsweise die Adsorption an einen inerten oder elektrisch geladenen anorganischen oder organischen Träger. Solche Träger können beispielsweise poröse Gele, Aluminiumoxid, Betonid, Agarose, Stärke, Nylon oder Polyacrylamid sein. Die Immobilisierung erfolgt hierbei durch physikalische Bindungskräfte, oft unter Beteiligung von hydrophoben Wechselwirkungen und ionischen Bindungen. Derartige Methoden sind vorteilhafterweise einfach zu handhaben und sie beeinflussen die Konformation der Peptide nur in geringem Umfang. Durch elektrostatische Bindungskräfte zwischen den geladenen Gruppen der Peptide und dem Träger kann die Bindung vorteilhafterweise verbessert werden, zum Beispiel durch die Verwendung von Ionenaustauschern, insbesondere Sephadex.

Ein weiteres Verfahren ist die kovalente Bindung an Trägermaterialen. Die Träger können dazu reaktive Gruppen aufweisen, die mit Aminosäure-Seitenketten homöopolare Bindungen eingehen. Geeignete Gruppen in Peptiden sind Carboxy-, Hydroxy- und Sulfidgruppen und insbesondere die endständigen Aminogruppen von Lysinen. Aromatische Gruppen bieten die Möglichkeit für Diazo-Kopplungen. Die Oberfläche von mikroskopischen porösen Glaspartikeln kann durch Behandlung mit Silanen aktiviert und anschließend mit Peptiden umgesetzt werde. Hydroxy-Gruppen natürlicher Polymere können zum Beispiel mit Bromzyan aktiviert und anschließend mit Peptiden gekoppelt werden. Mit Polyacrylamid-Harzen können zahlreiche Peptide vorteilhafterweise direkte kovalente Bindungen eingehen. Bei dem Einschluss in dreidimensionale Netzwerke werden die Peptide in ionotrophe Gele oder andere dem Fachmann bekannte Strukturen eingeschlossen. Die Poren der Matrix sind insbesondere so beschaffen, dass die Peptide zurückgehalten werden und eine Interaktion mit den Ziel-Molekülen möglich ist Bei der Quervernetzung werden die Peptide durch Vernetzung mit bifunktionellen Agenzien in polymere Aggregate umgewandelt. Derartige Strukturen sind gelatinös und leicht verformbar und insbesondere für den' Einsatz in verschiedenen Reaktoren geeignet. Durch Zugabe anderer inaktiver Komponenten, wie zum Beispiel Gelatine, bei der Vernetzung können die mechanischen und Bindungseigenschaften vorteilhafterweise verbessert werden. Bei der Mikroverkapselung wird der Reaktionsraum der Peptide mit Hilfe von Membranen eingegrenzt. Die Mikroverkapselung kann zum Beispiel als Grenzflächen-Polymerisation durchgeführt werden. Durch die Immobilisierung bei der Mikroverkapselung werden die Peptide unlöslich und dadurch wieder verwendbar. Im Sinne der Erfindung sind immobilisierte Peptide alle Peptide, die sich in einem Zustand befinden, der ihre Wiederverwendung erlaubt. Die Einschränkung der Beweglichkeit und der Löslichkeit der Peptide auf chemischem, biologischem oder physikalischem Wege führt vorteilhafterweise zu niedrigen Verfahrenskosten, insbesondere bei der Eliminierung von Autoantikörpern aus Blutbestandteilen.

In einer weiteren bevorzugten Ausführungsform der Erfindung ist das Peptid an eine Festphase gebunden. Die Bindung des Peptides an die Festphase kann über einen Spacer erfolgen. Als Spacer können alle chemischen Verbindungen eingesetzt werden, die für die Funktion des Spacers die geeigneten strukturellen und funktionellen Voraussetzungen aufweisen, solange sie nicht das Bindeverhalten derart modifizieren, dass eine Bindung des Autoantikörpers mit dem Peptid nachteilhafterweise beeinträchtigt wird.

Die Erfindung betrifft auch eine pharmazeutische Zusammensetzung, die die erfindungsgemäßen Peptide gegebenenfalls mit einem pharmazeutisch verträglichen Träger umfasst. Die pharmazeutische Zusammensetzung kann insbesondere als Arzneimittel ex vivo eingesetzt werden. Hierzu ist es beispielsweise möglich, die Peptide durch Zyklisierung oder andere dem Fachmann bekannte Verfahren so zu modifizieren, dass sie durch körpereigene peptidabbauende Strukturen, wie zum Beispiel Serumproteasen, nicht zerstört werden können. Durch Verwendung der erfindungsgemäßen Peptide ist es möglich, die Autoantikörper ex vivo zu neutralisieren. Bei einer ex vivo Neutralisation wird beispielsweise das Blut über eine Schleife - zum Beispiel in Form eines Schlauch-Kreislaufes - aus dem Körper geleitet, folgend mit dem Arzneimittel in Kontakt gebracht und nach der erfolgten Neutralisation der Autoantikörper wieder in den organismus, insbesondere dem Patienten, zurückgeführt. Im Sinne der Erfindung gelten als Arzneimittel sowohl solche pharmazeutischen Zusammensetzungen, die für die therapeutischen und prophylaktischen Zwecke ex vivo verwendet werden als auch solche pharmazeutischen Zusammensetzungen, die als Diagnostikum eingesetzt werden können.

Arzneimittel oder pharmazeutische Zusammensetzungen, die vorliegend synonym verwendet werden, sind erfindungsgemäß Stoffe und Zubereitungen aus Stoffen, die dazu bestimmt sind, durch ex vivo Anwendung Krankheiten, Leiden, Körperschäden oder krankhafte Beschwerden zu heilen, zu lindern oder zu verhüten. Medizinische Hilfsstoffe sind erfindungsgemäß solche Stoffe; die zur Produktion als aktive Ingredienzien von Arzneimitteln eingesetzt werden. Pharmazeutisch-technische Hilfsstoffe dienen der geeigneten Formulierung des Arzneimittels oder der pharmazeutischen Zusammensetzung und können sogar, sofern sie nur während des Herstellungsverfahrens benötigt werden, anschließend entfernt werden oder können als pharmazeutisch verträgliche Träger Teil der pharmazeutischen Zusammensetzung sein. Beispiele für pharmazeutisch verträgliche Träger sind nachstehend aufgeführt. Die Arzneimittelformulierung oder Formulierung der pharmazeutischen Zusammensetzung erfolgt gegebenenfalls in Kombination mit einem pharmazeutisch verträglichen Träger und/oder Verdünnungsmittel. Beispiele für geeignete pharmazeutisch verträgliche Träger sind dem Fachmann bekannt und umfassen zum Beispiel Phosphat-gepufferte Kochsalzlösungen, Wasser, Emulsionen wie zum Beispiel Öl/Wasser-Esmulsionen, verschiedene Arten von Detergenzien, sterile Lösungen, etc. Arzneimittel oder pharmazeutische Zusammensetzungen, die solche Träger umfassen, können mittels bekannter konventioneller Methoden formuliert werden. Diese Arzneimittel oder pharmazeutischen Zusammensetzungen können einem Individuum in einer geeigneten Dosis verabreicht werden, beispielsweise in einem Bereich von 1µg bis 10 g an Peptiden pro Tag und Patient. Bevorzugt werden dabei Dosen von 1 mg bis 1 g. Bevorzugt wird eine Verabreichung von möglichst wenigen und niedrigen Dosen und weiter bevorzugt eine einmalige Dosis. Die Verabreichung kann auf verschiedenen ex vivo Wegen erfolgena. Die Art der Dosierung und des Verabreichungsweges kann vom behandelnden Arzt entsprechend den klinischen Faktoren bestimmt werden..Es ist dem Fachmann bekannt, dass die Art der Dosierung von verschiedenen Faktoren abhängig ist, wie zum Beispiel der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden. Weiterhin ist dem Fachmann bekannt, dass er die Konzentration der Autoantikörper mit den erfindungsgemäßen Peptiden zunächst diagnostizieren kann, um die notwendige Konzentration des Arzneimittels zu bestimmen.

Die pharmazeutische Zusammensetzung oder das Arzneimittel kann selbstverständlich auch eine Kombination von zwei oder mehreren der erfindungsgemäßen pharmazeutischen Zusammensetzungen oder Arzneimittel sein, sowie eine Kombination mit anderen Arzneimitteln, wie beispielsweise Antikörpertherapien, Chemotherapien oder Radiotherapien, die auf eine geeignete Weise zeitlich gemeinsam oder getrennt verabreicht bzw. angewandt werden. Die Herstellung der Arzneimittel oder pharmazeutischen Zusammensetzungen erfolgt nach an sich bekannten Methoden.

Die Erfindung betrifft auch einen Kit umfassend das Peptid gegebenenfalls mit einer Anleitung oder Information zur pharmazeutischen Bereitstellung bzw. zum ex vivo therapeutischen Behandlungsverfahren. Die Information kann beispielsweise ein Beipackzettel sein oder ein anderes Medium, was dem Anwender Informationen darüber gibt, in welchem therapeutischen Verfahren die genannten Substanzen einzusetzen sind. Der Beipackzettel enthält insbesondere detaillierte und/oder wesentliche Informationen über das Heilverfahren. Selbstverständlich ist es nicht zwingend erforderlich, dass die Information einen Beipackzettel darstellt, es ist möglich, dass diese Information beispielsweise über das Internet mitgeteilt wird.

Die Erfindung betrifft auch eine Vorrichtung zur Chromatographie, die die erfindungsgemäßen Peptide umfasst.

In einer bevorzugten Ausführungsform sind die Peptide innerhalb des Chromatographiesystems an eine Festphase gebunden.

Die erfindungsgemäße Vorrichtung kann insbesondere dazu verwendet werden, die Autoantikörper aus Flüssigkeiten eines Patienten zu eliminieren bzw. die Autoantikörper zu neutralisieren. Dieses Verfahren ist dem Fachmann unter dem Begriff der Immunadsorption und der Apheresetherapie bekannt. Mit Hilfe der Immunadsorption werden Immunglobuline aus dem Blut des Patienten entfernt. Vorteilhafterweise kann diese Immunadsorptionsbehandlung stationär und ambulant durchgeführt werden. Es kann vorgesehen sein, dass die Vorrichtung, insbesondere der so genannte Adsorber, Bestandteil eines extrakorporalen Blutkreislaufes ist. Hierbei wird dem Patienten aus einem größeren Körpergefäß, insbesondere einer Armvene, kontinuierlich bzw. diskontinuierlich Blut entnommen und mittels Filtration oder Zentrifugation in einzelne Bestandteile, wie beispielsweise die zellulären und die humoralen Bestandteile, separiert. Ein wesentlicher Bestandteil des Blutes, das hierdurch gewonnen wird, ist insbesondere Blutplasma. Das Blutplasma kann vorteilhafterweise durch die erfindungsgemäße Vorrichtung geleitet und nach Adsorption der Autoantikörper zusammen mit den zuvor separierten Blutbestandteilen, insbesondere den zellulären Bestandteilen, dem Patienten zurückgegeben werden, insbesondere durch eine andere Arm- bzw. Beinvene. Es kann weiterhin vorgesehen sein, dass die Peptide an einer Sepharose-Matrix immobilisiert sind. Diese Matrix kann in einen Behälter gegeben werden, der ein Volumen von 10 bis 400 ml aufweist. Das Blutplasma des Patienten kann dann über diese Matrix geleitet werden, wobei die Autoantikörper binden und so aus dem Blutplasma eliminiert werden können. Dem Fachmann sind verschiedene Möglichkeiten bekannt, derartige festphasenfixierte Peptide bereitzustellen, beispielsweise in Form von (i) regenerationsfähigen Adsorptionssäulen, in Form von (ii) Doppelsäulen als auch in Form von (iii) Einmalsäulen. Die verschiedenen Spül- und Elutionslösungen, die eine hohe Effizienz der Behandlung ermöglichen, können durch den Fachmann problemlos durch Routineversuche ermittelt werden. Durch die Bereitstellung der erfindungsgemäßen Lehre, insbesondere der erfindungsgemäßen Peptide, sind dem Fachmann verschiedene Möglichkeiten offenbart, diese ex vivo zur Prophylaxe, Diagnose, Therapie als auch zur Nachbehandlung von Kälte-induzierten, Autoantikörper-vermittelten Krankheiten einzusetzen.

Die Erfindung betrifft auch ein Verfahren zur Behandlung einer Kälteallergie durch eine Bindung und/oder eine Entfernung von Autoantikörpern mittels von an eine Festphase gebundene erfindungsgemäße Peptide.

In einer bevorzugten Ausführungsform der Erfindung sind die Autoantikörper gegen PAR-1, PAR-2 und/oder PAR-3 gerichtet;

Die Erfindung betrifft auch die exo vivo Verwendung der erfindungsgemäßen der erfindungsgemäßen pharmazeutischen Zusammensetzung, des erfindungsgemäßen Kits als auch der erfindungsgemäßen Vorrichtung zur Prophylaxe, Diagnose, Therapie, Verlaufskontrolle und/oder Nachbehandlung von Durchblutungsstörungen.

In einer bevorzugten Ausführungsform der Erfindung sind die Durchblutungsstörungen eine Akrozyanose, eine Kältehämagglutinationskrankheit, ein Hyperviskositätssyndrom, ein Ischämiesyndrom, eine Akrotrophoneurose, eine Sklerodermie, ein Kälteerythem, eine Kältepurpura, eine Kälte-' urtikaria, eine Kryopathie und/oder eine Kryoglobulinämie. Eine Akrozyanose im Sinne der Erfindung ist jede Verfärbung der Akren bei allgemeiner Zyanose infolge örtlicher, venös-kapillärer vasomotorischer Störungen, die verstärkt bei Kälte und Nässe und mit Neigung zu Erfrierungen auftreten. Erfindungsgemäß ist die Akrozyanose insbesondere A. anaesthetica, A.e frigore und/oder A. juvenile. Eine Kältehämagglutinationskrankheit im Sinne der Erfindung ist eine erworbene Krankheit, die auf der Bildung von Kältehämagglutin beruht. Sie kann beispielsweise eine Hämolyse zur Folge haben, insbesondere wenn die Umgebungstemperatur auf unter 20 °C fällt. Das Hyperviskositätssyndrom ist erfindungsgemäß eine Krankheit, bei der durch erhöhte' Viskosität das Fließvermögen des Blutes herabgesetzt wird. Das Ischämiesyndrom wird im Sinne der Erfindung .in das akrale und das intermittierende Ischämiesyndrom unterschieden. Es werden hierunter im Sinne der Erfindung alle funktionellen, organischen und gemischt-funktionell organischen Durchblutungsstörungen der Hände und Füße verstanden. Im engeren Sinne kann es sich beispielsweise auch um das Raynaud-Syndrom handeln. Die Akrotrophoneurose bedeutet die Störung der Durchblutung der Gewebsernährung an den Gliedmaßen, insbesondere an den Gliedmaßenenden. Sklerodermie im Sinne der Erfindung ist ein Oberbegriff für chronisch verlaufende Krankheiten mit bindegewebiger Verhärtung entweder umschriebener Hautareale oder bei generalisiertem Befall der Haut unter Beteiligung innerer Organe. Im Sinne der Erfindung werden zum einen die diffuse oder progressive und zirkumskripte Sklerodermie unterschieden. Das Kälteerythem ist beispielsweise ein akuter Gewebsschaden durch eine Kälteeinwirkung, insbesondere infolge von Mangeldurchblutung, aber auch infolge eines direkten thermischen Angriffs. Hierbei kann es beispielsweise zu disköntinuierlichen Nervendegenerationen, Muskel- und Fettgewebsnekrosen als auch zu Knochenschäden kommen. Die Kältepurpura ist im Sinne der Erfindung eine kleine Blutung im Bereich der Haarfollikel infolge einer Kälteeinwirkung. Unter Kälteurtikaria wird die Quaddelbildung verstanden, nachdem ein Hautareal mit einem kalten Gegenstand, mit kaltem Wasser oder mit kaltem Wind in Kontakt gekommen ist. Eine Kryopathie ist im Sinne der Erfindung eine örtliche oder ein allgemeiner Krankheitszustand nach echter Kälteschädigung bzw. eine Krankheit mit Auftreten von Kälteglobolinen, bei konstitutioneller Kälteunverträglichkeit.

Nach einer weiteren bevorzugten Ausführungsform der Erfindung ist die Durchblutungsstörung eine Kälteallergie. Besonders bevorzugt ist die Kälteallergie ein Raynaud-Syndrom. Erfindungsgemäß wird zwischen dem Primär- und dem Sekundär-Raynaud-Syndrom unterschieden. Primär ist die Krankheit eine funktionelle Störung der kleinen versorgenden Gefäße der Akren ohne erkennbare Grunderkrankungen. Charakteristisch ist beispielsweise der beidseitige Befall - auch Symmetrie - der Hände bzw. der Zehen. Die Beschwerden der primären Krankheit lassen im Alter nach. Diese Krankheit kommt bei 20 % der Raynaud-Patienten vor. Die sekundäre Krankheit ist häufig ein ungleicher Befall beider Hände oder Füße als Ausdruck einer anderen zugrunde liegenden Erkrankung, teils ohne organische Gefäßerkrankungen, wie zum Beispiel Nervenschädigungen, bei bestimmten Medikamenten wie zum Beispiel Migränemitteln. Häufig ist die Krankheit mit einer organischen Gefäßerkrankung, vor allem einer Gefäßentzündung im Rahmen einer Bindegewebserkrankung, einer so genannten Kollagenose, verbunden. Beim Fortschreiten der Erkrankung kann es zu Wachstumsstörungen der Nägel sowie Absterben der Fingerkuppen (verhärtendes Phänomen) kommen.

Die Erfindung betrifft auch die Verwendung der erfindungsgemäßen Peptide des erfindungsgemäßen Kits und/oder der erfindungsgemäßen Vorrichtung zum Screenen von Arzneimitteln. Das Screenen von Arzneimitteln kann beispielsweise die Identifizierung von Substanzen, insbesondere Peptiden, Proteinen, Kohlenhydraten und/oder Lipiden umfassen, die mit den Peptiden und demgemäß mit den Loops, insbesondere dem zweiten Loop von PAR-1, PAR-2 und/oder PAR-3 wechselwirken.

Eine Wechselwirkung kann beispielsweise eine Bindung an diese Peptide sein oder aber auch eine Aktivierung bzw. eine Inhibierung von oder durch Peptide. Ein Arzneimittel könnte demgemäß beispielsweise eine Struktur sein, die im Körper eines Patienten an die Peptide, und dementsprechend an die Loops bindet und so mit den Autoanptikörpern um eine Bindungsstelle konkurriert. Durch die Offenbarung der erfindungsgemäßen Lehre, insbesondere über die Offenbarung des Zusammenhangs von Krankheit und dem Bindungsort der Autoantikörper, kann der Fachmann verschiedene Arzneimittel screenen. Das Screenen von Arzneimitteln aufgrund von offenbarten Targets gehört zum allgemeinen Wissen des Fachmanns und erfolgt durch Routineversuche; es sei auf die entsprechenden Standardwerke der Molekularbiologie und Pharmakologie verwiesen.'

Die Erfindung betrifft auch ein Verfahren zur Behandlung einer Autoimmunkrankheit durch eine Bindung und/oder Entfernung von Autoantikörpern mittels von an eine Festphase gebundenen erfindungsgemäßen Peptiden. Durch die an die Festphase gebundenen Peptide werden die Autoantikörper gebunden, komplexiert und/oder an der Festphase neutralisiert.

In einer bevorzugten Ausführungsform der Erfindung werden mit den genannten erfindungsgemäßen Stoffen und Erzeugnissen sowie den Vorrichtungen, insbesondere der Chromotographievorrichtung und den Peptiden gegen PAR-1, PAR-2 und/oder PAR-3 gerichtete Autoantikörper detektiert, gebunden, komplexiert und/oder neutralisiert. Beispielsweise können die Peptide dazu eingesetzt werden, mit einem ELISA oder einem anderen einem Fachmann bekannten immunologischen Detektionsverfahren, Autoantikörper in Seren von Patienten nachzuweisen. Für die Detektion kann es beispielsweise vorteilhaft sein, dass die Autoantikörper durch biotinylierte bzw. anderweitig gekoppelte Peptide gebunden und durch Streptavidin-gekoppelte Träger, wie zum Beispiel Magnetpartikel oder -platten, separiert werden. Ein solches Verfahren ist in der DE 102 56 897.9 beschrieben. Die separierten Autoantikörper werden insbesondere durch IgG-Subtyp-spezifische markierte Antikörper detektiert. Im Falle des Raynaud-Syndroms werden die Antikörper insbesondere mit IgG₁-Subtyp-spezifischen markierten Antikörpern detektiert.

Im Folgenden soll die Erfindung anhand von Beispielen näher erläutert werden, ohne auf diese'beschränkt zu sein:

### Beispiele

### I. Affinitätschromatographische Reinigung der Antikörper

Die biotinylierte Peptidsequenz LNITTCHD oder LNITTCHDCL der zweiten extrazellulären Schleife des PAR-2 Rezeptors wurde zur affinitätschromatographischen Reinigung der Autoantikörper gegen den PAR-2 Rezeptor eingesetzt. Da die angeführte Sequenz sowohl auf der zweiten extrazellulären Schleife des PAR-2 als auch des PAR-1 Rezeptors als Epitop der Autoantikörper identifiziert wurde, können mit dieser Sequenz Autoantikörper gegen den PAR-1, PAR-2 und auch gegen den PAR-3 Rezeptor gereinigt werden.

Aus den Patientenseren werden durch Ammoniumsulphatfällung und sich anschließender Dialyse die Immunglobulinfraktionen gewonnen. Diese IgG Fraktionen (1 ml) werden mit 300 *µ*l der Peptidlösung (100 *µ*g/ml) gemischt und 1 Stunde bei Raumtemperatur inkubiert. Das Gemisch, das den Antikörper - Peptidkomplex enthält, wird zu den zuvor 3 x mit phosphatgepufferten physiologischen Kochsalzlösung (PBS, pH 7,2) gewaschenen Streptavidin-Magnetpartikeln gegeben. Nach einer Stunde werden die Partikel im Magnetfeld separiert, 3x mit. PBS gewaschen und nach der letzten Separation werden die Antikörper mit 300 *µ*l einer 3 M KSCN Lösung von den Magnetpartikeln eluiert. Anschließend werden die Eluate gegen die PBS Lösung dialysiert. Anschließend werden die gereinigten PAR-Rezeptor Autoantikörper und die Immunglobulinfraktionen, aus denen die Antikörper entfernt wurden, hinsichtlich ihrer funktionellen Aktivität im Bioassay (spontan pulsierende Rattenkardio-myozyten) analysiert.

### II. Neutralisation der agonistischen PAR-Rezeptor Autoantikörper

Zur Neutralisation der funktionellen Autoantikörper werden synthetisierte Peptide, die der zweiten extrazellulären Schleife des PAR-1 (QTIQVPGLNITTCHDVLNETLL) bzw. des PAR-2 (QTIFIPALNITTCHDVLPEQLL) Rezeptors entsprachen, mit den aus den Seren von Patienten mit Raynoud's Syndrom präparierten Immunglobulinfraktionen inkubiert.

Die Inkubation erfolgte ebenfalls mit der als Antikörper-Epitop identifizierten Aminosäuresequenz LINTTCHDVL der zweiten extrazellulären Schleife des PAR-1 und PAR-2 Rezeptors. Die Immunglobulinfraktionen (50 *µ*l) wurden mit 50 *µ*l der Peptidlösung (10 *µ*g/ml) 1 Stunde bei Raumtemperatur inkubiert. Der Peptid - Antikörperkomplex wurde anschließend im Bioassay hinsichtlich seiner funktionellen Aktivität untersucht. Nach der Vorbehandlung der Antikörper mit den entsprechenden Peptiden war der agonistische Effekt aufgehoben.. Peptide, die der ersten extrazellulären Schleife.der PAR - Rezeptoren entsprachen bzw. der zweiten extrazellulären Schleife des betal adrenergen Rezeptors bzw. des Angiotensin II AT1 Rezeptors entsprachen, zeigten hinsichtlich der PAR Rezeptoren keine neutralisierende Wirkung.

## Patentansprüche

1. Peptid,
**dadurch gekennzeichnet, dass**
es ausgewählt ist aus der Gruppe bestehend aus:
a) einem Peptid bestehend aus der Aminosäuresequenz ITTCHDVL, ITTCHDAL und/oder LNITTCHD, oder
b) einem Peptid gemäß einer Aminosäuresequenz a), welches durch Deletionen, Substitutionen, Translokationen und/oder Inversionen modifiziert ist und die Sequenz des Peptids 60%, vorzugsweise 70%, bevorzugt 80%, ganz besonders bevorzugt 90% homolog zu einer der unter a) angegebenen Aminosäuresequenz ist, wobei
die Peptide mit einen an der zweiten extrazellulären Schleife eines Proteaseaktivierten Rezeptor (PAR), ausgewählt aus der Gruppe umfassend PAR-1, PAR-2 und/oder PAR-3, wechselwirkenden Autoantikörper binden für die ex vivo Verwendung zur Prophylaxe, Diagnose, Therapie, Verlaufskontrolle und/oder Nachbehandlung von mit Kälte-induzierten Autoantikörper-vermittelten Durchblutungsstörungen.

2. Peptid nach einem der vorhergehenden Ansprüche,
**dadurch gekennzeichnet, dass**
das Peptid immobilisiert ist.

3. Peptid nach einem der vorhergehenden Ansprüche,
**dadurch gekennzeichnet, dass**
das Peptid an eine Festphase gebunden ist.

4. Peptid nach einem der vorhergehenden Ansprüche
**dadurch gekennzeichnet, dass**
es zusätzlich Aminogruppen, Acetylgruppen, Biotingruppen, Spacer und/oder Linker umfasst, wobei die Spacer und/oder Linker ausgewählt aus der Gruppe bestehend aus Maleinamidocarbonsäure-Derivate; 4-Alkylphenyl-Derivate; 4-Oligoalkoxyphenyl- oder 4-Oligoalkoxyphenoxy-Derivate; 4-Oligoalkylmercaptophenyl- oder 4-Oligoalkylmercapto-phenoxy-Derivate; 4-Oligoalkylaminphenyl- oder 4-Oligoalkyl-aminphenoxy-Derivate; (Oligoalkylbenzyl)-phenyl- oder 4-(Oligo-alkylbenzyl)-phenoxy-Derivate sowie 4-(Oligoalkoxy-benzyl)-phenyl- oder 4-(Oligoalkoxybenzyt)-phenoxy-Derivate; Trityl-Derivate; Benzyloxyaryl- oder Benzyloxyalkyl-Derivate; Xanthen-3-yloxyalkyl-Derivate; (4-Alkylphenyl)- oder w-(4-Alkylphenoxy)-alkansäure-Derivate; Oligoalkoxy-phonxyalkyl-Derivate; Carbamat-Derivate; Trialkylsilyl- oder Dialkyl-akoxysilyl-Derivate.

5. Kit umfassend ein Peptid gemäß einem der Ansprüche 1 bis 4 gegebenenfalls mit einer Anweisung zum Kombinieren der Inhalte des Kits und/oder zum Bereitstellen einer Formulierung.

6. Vorrichtung zur Chromatographie umfassend Peptide gemäß einem der Ansprüche 1 bis 4.

7. Vorrichtung nach Anspruch 6,
**dadurch gekennzeichnet, dass**
die Peptide an eine Festphase gebunden sind.

8. Ex-vivo Verwendung ein Peptid gemäß einem der Ansprüche 1 bis 4, eines Kits nach Anspruch 5, einer Vorrichtung nach Anspruch 6 oder 7 zur Prophylaxe, Diagnose, Therapie, Verlaufskontrolle und/oder Nachbehandlung von mit Kälte-induzierten Autoantikörper-vermittelten Durchblutungsstörungen.

9. Verwendung nach Anspruch 8,
**dadurch gekennzeichnet, dass**
die Durchblutungsstörung eine Akrozyanose, eine Kältehämaglutinationskrankheit, ein Hyperviskositätssyndrom, ein Ischämiesyndrom, eine Akrotrophoneurose, eine Sklerodermie, ein Kälteerythem, eine Kältepurpura, eine Kälteurtikaria, eine Kryopathie und/oder eine Kryoglobulinämie ist.

10. Verwendung nach einem der Ansprüche 8 oder 9,
**dadurch gekennzeichnet, dass**
die Durchblutungsstörung eine Kälteallergie ist.

11. Verwendung nach Anspruch 10,
**dadurch gekennzeichnet, dass**
die Kälteallergie ein Raynaud-Syndrom ist.

12. Verwendung nach einem der Ansprüche 8 bis 11,
**dadurch gekennzeichnet, dass**
gegen PAR-1, PAR-2 und/oder PAR-3 gerichtete Autoantikörper detektiert, gebunden, kompiexiert und/oder neutralisiert werden.

## Claims

1. A peptide,
**characterised in that**
it is selected from the group consisting of:
a) a peptide consisting of the amino acid sequence ITTCHDVL, ITTCHDAL and/or LNITTCHD, or
b) a peptide according to an amino acid sequence a), which is modified by deletions, substitutions, translocations and/or inversions with the sequence of said peptide being 60%, preferably 70%, preferably 80%, most preferably 90% homologous to one of the amino acid sequences specified under a), wherein
the peptides bind to an antibody that interacts with the second extracellular loop of a protease-activated receptor (PAR), selected from the group consisting of PAR-1, PAR-2 and/or PAR-3, for ex vivo use for prophylaxis, diagnosis, therapy, follow-up and/or post-treatment of cold-induced autoantibody-induced circulatory disorders.

2. The peptide according to any one of the preceding claims,
**characterised in that**
the peptide is immobilised.

3. The peptide according to any one of the preceding claims,
**characterised in that**
the peptide is bound to a solid phase.

4. The peptide according to any one of the preceding claims
**characterised in that**
it additionally comprises amino groups, acetyl groups, biotin groups, spacers and/or linkers, wherein the spacers and/or linkers are selected from the group consisting of maleimide carboxylic acid derivates; 4-alkylphenyl derivates; 4-oligoalkoxyphenyl or 4-oligoalkoxyphenoxy derivatives; 4-oligoalkylmercaptophenyl or 4-oligoalkylmercaptophenoxy derivatives; 4-oligoalkylaminophenyl or 4-oligoalkylaminophenoxy derivatives; (oligoalkylbenzyl)-phenyl or 4-(oligoalkylbenzyl)-phenoxy derivatives, as well as 4-(oligoalkoxybenzyl)-phenyl or 4-(oligoalkoxybenzyl)-phenoxy derivatives; trityl derivatives; benzyloxyaryl or benzyloxyalkyl derivatives; xanthene-3-yloxyalkyl derivatives; (4-alkylphenyl)- or w-(4-alkylphenoxy)-alkanoic acid derivatives; oligoalkoxyphenoxyalkyl derivatives; carbamate derivatives; trialkylsilyl or dialkylalkoxysilyl derivatives.

5. A kit comprising the peptide according to any one of claims 1 to 4 optionally with instructions to combine the contents of the kit and/or to provide a formulation.

6. A device for chromatography comprising peptides according to any one of claims 1 to 4.

7. The device according to claim 6,
**characterised in that**
the peptide is bound to a solid phase.

8. Ex vivo use of the peptide according to any one of claims 1 to 4, the kit according to claim 5, the device according to claim 6 or 7 for prophylaxis, diagnosis, therapy, follow-up and/or post-treatment of cold-induced autoantibody-induced circulatory disorders.

9. The use according to claim 8,
**characterised in that**
the circulatory disturbance is an acrocyanosis, a cold hemagglutination disease, a hyperviscosity syndrome, an ischemia syndrome, an acrotrophoneurosis, a scleroderma, a cold erythema, a cold purpura, a cold urticaria, a cryopathy and/or a cryoglobulinemia.

10. The use according to any one of claims 8 or 9,
**characterised in that**
the circulatory disorder is a cold allergy.

11. The use according to claim 10,
**characterised in that**
the cold allergy is Raynaud's syndrome.

12. The use according to any one of claims 8 to 11,
**characterised in that**
autoantibodies directed against PAR-1, PAR-2 and/or PAR-3 are detected, bound, complexed and/or neutralised.

## Revendications

1. Peptide
**caractérisé en ce qu'**il est
sélectionné du groupe composé de :
a) un peptide composé d'une séquence en acides aminés ITTCHDVL, ITTCHDAL et/ou LNITTCHD, ou
b) un peptide selon la séquence en acides aminés a), qui est modifié par délétions, substitutions, translocations et/ou inversions et la séquence du peptide est à 60%, de préférence à 70%, de préférence à 80% et de préférence particulière à 90% homologue à une des séquences en acides aminés mentionnés sous a),
les peptides se liant à un auto-anticorps interagissant sur la deuxième boucle extracellulaire d'un récepteur activé par des protéases (PAR), sélectionné du groupe comportant PAR-1, PAR-2 et/ou PAR-3, pour l'utilisation ex vivo pour la prophylaxie, le diagnostic, la thérapie, le contrôle de l'évolution et/ou le traitement ultérieur des troubles de la circulation sanguine causés par des auto-anticorps induits par le froid.

2. Peptide selon l'une quelconque des revendications susmentionnées,
**caractérisé en ce que**
le peptide est immobilisé.

3. Peptide selon l'une quelconque des revendications susmentionnées,
**caractérisé en ce que**
le peptide est lié à une phase solide

4. Peptide selon l'une quelconque des revendications susmentionnées,
**caractérisé en ce qu'**il
comporte également des groupes aminés, groupes d'acétyles, des groupes de biotines, des espaceurs et/ou liaisons, les espaceurs et/ou liaisons étant sélectionnés du groupe composé des dérivés d'acide amido-maléique carboxylique, dérivés 4 alcyle phényle, dérivés 4 oligo-alkoxy-phényle ou 4 oligo-alkoxy-phénoxy ; dérivés 4 oligo-alkyle-mercapto-phényle ou 4 oligo-alkyle-mercapto-phénoxy ; dérivés 4 oligo-alkyle-aminophényle ou 4 oligo-alkyle-aminophénoxy ; dérivés (oligo-alkyle-benzyle)-phényle ou 4 (oligo-alkyle-benzyle)-phénoxy ainsi que dérivés 4 (oligo-alkoxy-benzyle)-phényle ou 4 (oligo-alkoxy-benzyte ; dérivés trityle ; dérivés benzyle oxyariles ou benzyle-oxyalkyle ; dérivés xanthen-3-yloxy-alkyle ; dérivés (4-alkyle-phényle) ou w-(4-alkyle-phénoxy)-acide alcanique ; dérivés oligo-alkoxy-phonxy-alkyle, dérivés carbamate ; dérivés trialkylsilyle ou dialkyle-alkoxysilyle.

5. Kit comportant un peptide selon l'une quelconque des revendications 1 à 4, le cas échéant avec une instruction de combiner les contenus du kit et/ou de préparer une formule.

6. Dispositif de chromatographie comportant des peptides selon l'une quelconque des revendications 1 à 4.

7. Dispositif selon la revendication 6,
**caractérisé en ce que**
les peptides sont liés à une phase solide.

8. Utilisation ex vivo d'un peptide selon l'une quelconque des revendications 1 à 4, d'un kit selon la revendication 5, d'un dispositif selon la revendication 6 ou 7 pour la prophylaxie, le diagnostic, la thérapie, le contrôle de l'évolution et/ou le traitement ultérieur des troubles de la circulation sanguine causés par des auto-anticorps induits par le froid.

9. Utilisation selon la revendication 8,
**caractérisée en ce que**
le trouble de la circulation sanguine est une acrocyanose, une maladie d'hémagglutination induite par le froid, un syndrome d'hyperviscosité, un syndrome d'ischémie, un actrotrophonevrose, une sclérodermie, un érythème au froid, un purpura au froid, une urticaire au froid, une cryopathie et/ou une cryoglobulinémie.

10. Utilisation selon l'une quelconque des revendications 8 ou 9,
**caractérisée en ce que**
le trouble de la circulation sanguine est une allergie au froid.

11. Utilisation selon la revendication 10,
**caractérisée en ce que**
l'allergie au froid est un syndrome de Raynaud.

12. Utilisation selon l'une quelconque des revendications 8 à 11,
**caractérisée en ce que**
les auto-anticorps dirigés contre PAR-1, Par-2 et/ou PAR-3 sont détectés, liés, complexés et neutralisés.
